# EUROPEAN PATENT SPECIFICATION

(11) **EP 3 187 595 B1**
(45) Date of publication and mention of the grant of the patent: **16.01.2019**
(21) Application number: 15382680.5
(22) Date of filing: 30.12.2015
(51) Int. Cl.: C12Q 1/68, B01L 3/00

(54) **METHOD FOR THE DETECTION OF LEGIONELLA SPP. STRAINS IN ENVIRONMENTAL SAMPLES BASED ON LOOP-MEDIATED ISOTHERMAL AMPLIFICATION (LAMP), DETECTION REAGENT AND SET OF PRIMERS.**
VERFAHREN ZUM NACHWEIS VON LEGIONELLA SPP. STÄMME IN UMWELTPROBEN AUF BASIS VON LOOP-VERMITTELTER ISOTHERMALER AMPLIFIKATION (LAMP), NACHWEISREAGENS UND PRIMERSETS.
PROCÉDÉ POUR LA DÉTECTION DE LEGIONELLA SPP. SOUCHES DANS DES ÉCHANTILLONS ENVIRONNEMENTAUX SUR LA BASE D'UNE AMPLIFICATION ISOTHERME BASÉE SUR UNE BOUCLE (LAMP), RÉACTIF DE DÉTECTION ET ENSEMBLE D'AMORCE.

(43) Date of publication of application: 05.07.2017
(73) Proprietor: Fundación Gaiker, 48170 Zamudio Vizcaya (ES)
(72) Inventor: VERDOY BERASTEGUI, Dolores, 48170 Zamudio- Vizcaya (ES); OLABARRIA DE PABLO, Garbiñe, 48170 Zamudio- Vizcaya (ES)
(74) Representative: Balder IP Law, S.L.

(56) References cited:
- WO-A1-98/51693
- JP-A- 2003 219 878
- US-A1- 2011 104 692
- US-A1- 2015 037 803
- XI LU ET AL: "LAMP-based method for a rapid identification ofspp. and", APPLIED MICROBIOLOGY AND BIOTECHNOLOGY, SPRINGER, BERLIN, DE, vol. 92, no. 1, 17 August 2011 (2011-08-17), pages 179-187, XP019950899, ISSN: 1432-0614, DOI: 10.1007/S00253-011-3496-8
- "Loopamp TM Legionella Detection Kit E", , 1 March 2013 (2013-03-01), pages 1-2, XP055273060, Retrieved from the Internet: URL:http://loopamp.eiken.co.jp/e/products/ legionella_e/img/01.pdf [retrieved on 2016-05-17]
- "Final report Summary-PINVIALEG (Portable microfluidic-based device for in situ detection of viable legionella)", , 17 June 2013 (2013-06-17), XP055273577, Retrieved from the Internet: URL:http://cordis.europa.eu/result/rcn/583 18_en.pdf [retrieved on 2016-05-19]
- CHIH-HUNG WANG ET AL: "A magnetic bead-based assay for the rapid detection of methicillin-resistant Staphylococcus aureus by using a microfluidic system with integrated loop-mediated isothermal amplification", LAB ON A CHIP: MINIATURISATION FOR CHEMISTRY, PHYSICS, BIOLOGY, MATERIALS SCIENCE AND BIOENGINEERING, vol. 11, no. 8, 11 March 2011 (2011-03-11) , page 1521, XP55000530, GB ISSN: 1473-0197, DOI: 10.1039/c0lc00430h -& Chih-Hung Wang ET AL: "SUPPLEMENTARY INFORMATION- A magnetic bead-based assay for the rapid detection of methicillin- resistant Staphylococcus aureus by using a microfluidic system with integrated loop-mediated isothermal amplification", Lab Chip, 11 March 2011 (2011-03-11), pages 1-3, XP055274161, Retrieved from the Internet: URL:http://www.rsc.org/suppdata/lc/c0/c0lc 00430h/c0lc00430h.pdf [retrieved on 2016-05-20]
- BEJ A K ET AL: "DETECTION OF VIABLE LEGIONELLA PNEUMOPHILA IN WATER BY POLYMERASE CHAIN REACTION AND GENE PROBE METHODS", APPLIED AND ENVIRONMENTAL MICROBIOLOGY, AMERICAN SOCIETY FOR MICROBIOLOGY, US, vol. 57, no. 2, 1 February 1991 (1991-02-01), pages 597-600, XP008002032, ISSN: 0099-2240
- DATABASE Genbank [Online] 3 February 2015 (2015-02-03), "Legionella pneumophila strain Philadelphia 1 16S ribosomal RNA gene, complete sequence", XP002757984, retrieved from NCBI accession no. NR_074231.1 Database accession no. NR_074231.1
- SHAH UDDIN ET AL: "A Portable Automatic Endpoint Detection System for Amplicons of Loop Mediated Isothermal Amplification on Microfluidic Compact Disk Platform", SENSORS, vol. 15, no. 3, 5 March 2015 (2015-03-05), pages 5376-5389, XP055273914, DOI: 10.3390/s150305376

## Description

### FIELD OF THE INVENTION

The present invention relates to a method based on loop-mediated isothermal amplification (LAMP) designed to amplify specifically sequences of *Legionella's* 16S ribosomal RNA (rRNA) gene using a set of specially designed LAMP primers, to a detection reagent for specifically detecting sequences of *Legionella's* 16S rRNA gene, to a set of LAMP primers to amplify specific regions of *Legionella's* 16S rRNA gene.

### BACKGROUND OF THE INVENTION

The *Legionella* outbreaks have a great Sanitary, Economic and Social impact, with yearly cases of seriously infected individuals, and deceases. Therefore, the Industrial sectors involved and the Administration, are deploying a great effort with the aim to reinforce the prevention measures as well as the control of *Legionella* environmental contamination.
Legionnaires' disease is a form of pneumonia caused by the bacterium *Legionella pneumophila* and related bacteria. A less severe form of the disease is a respiratory infection called Pontiac fever. Legionnaires' disease is normally contracted by inhaling tiny droplets of water (aerosols), contaminated with *Legionella.* However, most people exposed to *Legionella* do not become ill and person-to-person spread of the disease has not been documented. Some people are at higher risk of contracting Legionnaires' disease, e.g. those over 45, smokers, heavy drinkers, people suffering from chronic respiratory or kidney disease, and people with immunosuppression. Legionnaires' disease not only affects the general public, e.g. travellers staying in hotels, but also workers, especially maintenance technicians of air-conditioning or water supply systems. There is evidence that workers in places where mist machines are present, dental practitioners, workers on off shore oil and gas installations, welders, vehicle washers, miners, healthcare workers, workers in industrial wastewater treatment plants, and other different industrial areas that involve water streams in their industrial processes, e.g. pulp and paper mills, which can also be exposed to *Legionella.* Risk systems for *Legionella* exposure include: water systems incorporating a cooling tower, water systems incorporating an evaporative condenser, hot and cold water systems, Spa pools (also known as whirlpool baths, hot tubs and spa baths), humidifiers and water misting systems, waterlines to dental chairs, aeration ponds in biological treatment plants and industrial wastewater treatment plants, high-pressure water cleaning machines, other plants and systems containing water which is likely to exceed 20°C in temperature and which may release a spray or aerosol.

The risks from exposure to *Legionella* are normally controlled by measures which prevent the proliferation of the bacteria in the system, and by reducing exposure to water droplets and aerosol. Precautions include: controlling the release of water sprays; avoiding water temperatures between 20°C and 45°C; avoiding water stagnation that can encourage the growth of biofilm; avoiding the use of materials which harbour bacteria and other microorganisms or provide nutrients for microbial growth; maintaining cleanliness of the system and the water in it. As a last resort, maintenance personnel might need to use personal protection equipment (such as respirators).

Official methods for *Legionella* detection are based on the growth of the microorganism in selective media, it is based on ISO 11731 standard. At least 7 to 15 days are required for obtaining results due to the slow growth rate of the bacterium. Culture detection also shows low sensitivity, loss of viability of bacteria after collection, difficulty in isolating *Legionella* in samples contaminated with other microbial and the inability to detect viable non culturable bacteria. This test time lapse, is considered excessive and costly by the industrial maintenance sector.

Therefore, the development of a rapid and specific detection method for *Legionella infective strains* monitoring and in real time would be crucial for the efficient prevention of legionellosis. Immunodetection and Polymerase chain reaction (PCR) methods, have been described as useful tools for *L. pneumophila* detection. In fact, the legislation is open to the usage of other detection methods as Polymerase Chain Reaction (PCR).

In order to increase the efficiency and the cost effectiveness of the prevention and control labour against *Legionella,* there is a clear demand for rapid detection systems, portable, and easy to use; which will allow the rapid implementation of corrective actions, at the very first presence signals of this bacterium. Moreover, these smarter systems will provide the chance to perform on site surveillance of the biocides treatment, thus allowing the dosification variation and the biocidal compound, to eliminate the focus of a potential outbreak.

There are basically three alarm levels for *Legionella* that have to be assessed by the maintenance service. A risk alarm level: when the bacterium counting is >10,000 (colony forming units (CFU)/1000 ml); a control operations required level: when the bacterium counting is 1,000 (CFU/1000 ml); and a follow-up level: when the bacterium counting is 100 (CFU/1000 ml).

Different rapid solutions for *Legionella* detection have been developed in the last years. Among them the PCR systems, taking into account the detection limit and rapidness, are the most promising.

However, they still have a number of problems not properly resolved.

Conventional culture ISO 11731 delivers almost 60% of false negatives (Diaz-Flores A, Montero JC, Castro FJ, et al. Comparing methods of determining Legionella spp. in complex water matrices. BMC Microbiology. 2015;15:91. doi:10.1186/s12866-015-0423-7), mostly due to non-detection of viables non-culturable; high threshold of detection level; inhibition of *Legionella* growth in heavily contaminated (microbiological) samples; inhibition of *Legionella* growth in water systems with high content of dispersants, and metal traces. On the other hand, the current rapid genetic tests do not distinguish between viable, and non-viable death *Legionella* cells and deliver less than 10% of false positives (Diaz-Flores A, Montero JC, Castro FJ, et al. Comparing methods of determining Legionella spp. in complex water matrices. BMC Microbiology. 2015;15:91.doi:10.1186/s12866-015-0423-7). In both cases require skilled personnel and a microbiology Lab, and in case of the genetic tests a further molecular equipped lab is required.

On the other hand, it can happen discrepancies between PCR and culture results; which can be explained by several factors. *Legionella* growth can be inhibited or masked by overgrowth of contaminating microorganisms. Furthermore, *L. pneumophila* becomes non-culturable in biofilms in doses of 1 mg/L of monochloramine, making culture detection of this pathogen ineffective. The effectiveness of treatments on *Legionella pneumophila* (chlorine, heat, ozone, UV, monochloramine) has been mainly evaluated based simply on cultivability and that could not be a real indicative of the absence of intact viable cells *L. pneumophila* can enter a viable but noncultivable (VBNC) state, from which it can recover after passage in amoebae. These VBNC legionellae may be detected by PCR, along with dead bacteria, possibly explaining, at least in part, why PCR values are usually higher than those obtained by culture.

Other alternative rapid methods are the ones based on immuno-detection, which have provided the basis for the development of powerful analytical tools for a wide range of targets. During the last years, the number of publications in this field has increased significantly. Traditionally, the most common method applied to microorganism detection has been the enzyme-linked immunosorbent assay (ELISA). The main drawback of ELISA is the high detection limit generated; which is often between 10⁵ and 10⁶ CFU/mL. This limit may be improved to 103 and 104 cells/mL using more sensitive detection methods. The immobilization of antibodies onto the surface of magnetic beads to obtain immunomagnetic beads (IMB) has promoted the development of immunomagnetic separation (IMS). Thereby, IMS provides a simple but powerful method for specific capture, recovery and concentration of the desired microorganism from heterogeneous bacterial suspension, however, still on the edge of the required detection limit for environmental samples; and higher than the ones based on molecular procedures.

Most of the commercially available tests are based on molecular technology, which uses Legionella's biomarkers. The procedure is based on the recognition of specific sequences of the bacterium's DNA, its amplification by temperature cycles 60°C -72°C; and ulterior optical reading of fluorescence signals, by sophisticated optical, non-portable equipment. However, these kits are addressed for their usage in molecular labs and to be handled by qualified personnel.

More recently, new portable solutions based on immunodetection technologies have appeared on the market. For example, Hydrosense™, addressed to the maintenance sector, is based on immuno-detection technology. Its main drawbacks are: it only detects 1 out of the total 15 serogroups of *Legionella pneumophila;* it does not detect the other 18 groups of *non-pneumophilic Legionella* harmful for humans; and it does not distinguish between alive and death cells. Legipid® is also based on immune-detection, but is very complex to handle on site and, therefore not user friendly. Furthermore, the system only detects *Legionella pneumophila,* letting without control other 18 strains of *non-pneumophilic Legionellas,* which are virulent and harmful. This system is, however, able to distinguish between alive and death cells.

Thus, in view of the above there is still the need in the art to provide a new simple, rapid and user-friendly method for the detection of *Legionella* spp. in environmental samples that is capable of distinguishing between dead and alive cells and detecting as many infective *Legionella* strains as possible with a high level of sensitivity and specificity.

### SUMMARY OF THE INVENTION

The problem to be solved by the present invention is, to provide a simple, rapid and user-friendly method for the detection of infective *Legionella* spp. in environmental samples that is capable of distinguishing between dead and alive cells and detecting as many *Legionella* strains as possible with a high level of sensitivity, precision and specificity.

The solution is based on that the present inventors have identified that by amplifying specifically sequences of *Legionella's* 16S rRNA gene at a constant temperature with a specific blend of reagent solutions, comprising set of specially designed LAMP primers, it was possible to distinguish between alive and dead cells and to detect specifically 38 *Legionella* strains among 18 serogroups, at a sensitivity and specificity threshold from 5-100 Genomic Equivalent to at least 100 forming units of *Legionella,* in a simple, rapid and user friendly handling.

Accordingly, a first aspect of the invention relates to a method for the detection of *Legionella* spp. strains in an environmental sample based on loop-mediated isothermal amplification characterized in that it comprises the steps of:
(a) sample collection;
(b) genetic material extraction of the sample with an extraction reagent comprising at least cellular lysis reagents and a magnetizable support solution;
(c) elution of the genetic material from the magnetizable support, and isothermal RNA retrotranscription and cDNA amplification with a detection reagent comprising at least elution, rectrotranscriptase, amplification and reading solutions, wherein the amplification solution comprises a set of LAMP primers to amplify specific regions of *Legionella's* 16S rRNA gene; and
(d) result reading, wherein the set of LAMP primers to amplify specific regions of *Legionella's* 16S rRNA gene in step (c) comprises at least: a forward primer F3 comprising a nucleotide sequence as set forth in SEQ ID No. 1; a forward primer F2 comprising a nucleotide sequence as set forth in SEQ ID No. 2; a forward primer F1c comprising a nucleotide sequence as set forth in SEQ ID No. 3; a backward primer B3 comprising a nucleotide sequence as set forth in SEQ ID No. 4; a backward primer B2 comprising a nucleotide sequence as set forth in SEQ ID No. 5; a backward primer B1c comprising a nucleotide sequence as set forth in SEQ ID No. 6; a forward inner primer FIP comprising a nucleotide sequence as set forth in SEQ ID No. 7; and a backward inner primer BIP comprising a nucleotide sequence as set forth in SEQ ID No. 8.

In a second aspect, the invention relates to a detection reagent for specifically detecting sequences of *Legionella's* 16S rRNA gene, characterized in that it comprises at least elution, rectrotranscriptase, amplification and reading solutions, wherein the amplification solution comprises a set of LAMP primers to amplify specific regions of *Legionella's* 16S rRNA gene, and wherein the set of LAMP primers to amplify specific regions of *Legionella's* 16S rRNA gene comprises at least: a forward primer F3 comprising a nucleotide sequence as set forth in SEQ ID No. 1; a forward primer F2 comprising a nucleotide sequence as set forth in SEQ ID No. 2; a forward primer F1c comprising a nucleotide sequence as set forth in SEQ ID No. 3; a backward primer B3 comprising a nucleotide sequence as set forth in SEQ ID No. 4; a backward primer B2 comprising a nucleotide sequence as set forth in SEQ ID No. 5; a backward primer B1c comprising a nucleotide sequence as set forth in SEQ ID No. 6; a forward inner primer FIP comprising a nucleotide sequence as set forth in SEQ ID No. 7; and a backward inner primer BIP comprising a nucleotide sequence as set forth in SEQ ID No. 8.

In a third aspect, the invention related to a set of LAMP primers to amplify specific regions of *Legionella's* 16S rRNA gene, characterized in that it comprises at least comprises at least: a forward primer F3 comprising a nucleotide sequence as set forth in SEQ ID No. 1; a forward primer F2 comprising a nucleotide sequence as set forth in SEQ ID No. 2; a forward primer F1c comprising a nucleotide sequence as set forth in SEQ ID No. 3; a backward primer B3 comprising a nucleotide sequence as set forth in SEQ ID No. 4; a backward primer B2 comprising a nucleotide sequence as set forth in SEQ ID No. 5; a backward primer B1c comprising a nucleotide sequence as set forth in SEQ ID No. 6; a forward inner primer FIP comprising a nucleotide sequence as set forth in SEQ ID No. 7; and a backward inner primer BIP comprising a nucleotide sequence as set forth in SEQ ID No. 8.

The method for the detection of *Legionella* according to the invention is a molecular technique that has the advantages of being simple and rapid because of the simplification of the concentrations steps by usage of magnetic means to capture the analyte, thus avoiding spinning steps and the inhibitory compounds which could occur in the environmental samples. Moreover, it allows the simplification of the heating procedure, since it is carried out at a unique temperature without the requirement of temperature cycles. Furthermore, the method is capable of distinguish between alive and dead cells, since the detection is based on RNA biomarkers associated to the metabolism of living cells, instead of DNA biomarkers common in both dead and alive cells. Further, the reading of the results is simplified, due to the implementation in the procedure of fluorophores that can be detected by naked eye, without requiring sophisticated optical reading means.

The foregoing and other advantages of the present invention will be more clearly understood from the detailed description below of preferred embodiments provided only by way of an illustrative and non-limiting examples.

### DRAWINGS

FIG. 1: Nucleotide sequence of *Legionella pneumophila* subsp. *pneumophila* strain Philadelphia 1 16S ribosomal RNA gene, NCBI Reference Sequence: NR_074231.1>gi|444303809|ref|NR_074231.1|.
FIG. 2: Nucleotide sequence of the set of LAMP primers to amplify specific regions of *Legionella's* 16S rRNA gene.
FIG. 3: Nucleotide sequence of the Loop-primers to amplify specific regions of *Legionella's* 16S rRNA gene.
FIG. 4: Specificity results of the described LAMP method. Assay performed with genome samples, calibrated with the set of primers specified by the present invention; results shown, the obtained data from quantification cycle (Cq), or time in minutes in which the fluorescence emission, becomes exponential.
FIG. 5: Specificity results of the described LAMP method. Assay performed with genome samples, calibrated with a comparative set of primers specified by the present invention; results shown, the obtained data from quantification cycle (Cq), or time in minutes in which the fluorescence emission, becomes exponential. Below, the fluorescence emission of each reaction tubes, at the assay final time.

### DETAILED DESCRIPTION OF THE INVENTION

### Method for the detection of Legionella spp. in an environmental sample

### (a) Sample collection

Virtually all known environmental samples from different facilities can be analysed by means of the method according to the invention.

### (b) Extraction of the genetic material of the sample with an extraction reagent comprising cellular lysis reagents and a magnetizable support solution

In an embodiment of the invention the extraction reagent according to the invention comprises at least one enzyme suitable for bacterial lysis, at least one washing buffer and at least one chelating agent.

In principle, any enzyme capable of cellular lysis may be used. Suitable enzymes according to the invention comprise, but are not limited to: glycoside hydrolases, proteinase K, muramidases, lysozyme. In a preferred embodiment of the invention a wide spectrum glycoside hydrolase is used.

In a preferred embodiment the at least one enzyme capable of cellular lysis is comprised in an amount between 5 to 40 mg/ml.

Suitable washing buffers according to the invention comprise but are not limited to Tris-HCl buffers.

In a preferred embodiment the washing buffer is comprised at a concentration between 5 to 20 mM, and with a pH from 5.5 to 8.5.

Examples of the most preferred chelating agents according to the invention are: DTPA (Diethylenetriaminepentaacetic acid; Diethylenetriamine-N,N,N',N',N"-pentaacetic acid; Pentetic acid; N,N-Bis(2-(bis-(carboxymethyl)amino)ethyl)-glycine; Diethylenetriamine pentaacetic acid, [[(Carboxymethyl)imino]bis(ethylenenitrilo)]-tetra-acetic acid; EDTA (Edetic acid; Ethylenedinitrilotetraacetic acid; EDTA, free base; EDTA free acid; Ethylenediamine-N,N,N',N'-tetraacetic acid; Hampene; Versene; N,N'-1,2-Ethane diylbis-(N-(carboxymethyl)glycine); ethylenediamine tetra-acetic acid; NTA (N,N-bis(carboxymethyl)glycine; Triglycollamic acid; Trilone A; alpha,alpha',alpha"-trimethylaminetricarboxylic acid; Tri(carboxymethyl)amine; Aminotriacetic acid; Hampshire NTA acid; nitrilo-2,2',2"-triacetic acid; Titriplex i; Nitrilotriacetic acid.

In a preferred embodiment the chelating agent is comprised at a concentration between 0.1 mM to 10 mM.

After the *Legionella* spp. cells are ruptured the nucleic acids are released and captured by the magnetizable support solution present in the extraction reagent. The nucleic acids are retained on the surface of said magnetizable support and, thus, separated from the rest cell debris and liquid waste.

Accordingly, in principle any magnetizable support capable of trapping nucleic acids may be used according to the invention.

In an embodiment the magnetizable support comprises magnetic micro-beads.

In a preferred embodiment the extraction reagent in step (b) comprises: 5 to 40 mg/ml of at least a wide spectrum glycoside hydrolase, a washing buffer comprising at least Tris-HCl at a concentration between 5 to 20 mM and pH from 5.5 to 8.5, and at least one chelating agent at a concentration between 0.1 mM to 10 mM.
(c) elution of the genetic material from the magnetizable support, and isothermal RNA retrotranscription and cDNA amplification with a detection reagent comprising at least elution, rectrotranscriptase, amplification and reading solutions, wherein the amplification solution comprises a set of LAMP primers to amplify specific regions of *Legionella's* 16S rRNA gene

The method for the detection of *Legionella* spp. according to the present invention is based on loop-mediated isothermal amplification (LAMP). Consequently, according to the invention, the isothermal amplification of specific regions of *Legionella's* 16S rRNA gene occurs at a constant temperature. This allows carrying out the molecular assay at a unique temperature without the requirement of temperature cycles.

In a preferred embodiment of the method according to the invention, the isothermal RNA retrotranscription and cDNA amplification in step (c) takes place simultaneously in the same reaction chamber at a temperature between 58°C and 74°C.

Another achievement of the method according to the present invention is the reagents cocktail that allows to carry out the retrotranscription and amplification steps simultaneously and in the same place, as well as reaching a sensitivity and specificity threshold from at least 5-100 genomic equivalent to at least 100 forming units of *Legionella* spp.

Suitable elution solutions according to the invention are those capable of eluting the genetic material from the magnetizable support. Examples comprise, but are not limited to protein solutions, e.g., bovine serum albumin (BSA) and/or casein, tris hydroxymethyl aminomethane (TRIS), Sodium dodecyl sulfate (SDS).

Suitable rectrotranscriptase solutions according to the invention are those that allow the synthesis of DNA from RNA by a process of reverse transcription.

In an embodiment, the rectrotranscriptase solutions is a retrotranscriptase buffer comprising at least an isothermal rectrotransciptase and at least one cofactor.

In one embodiment, the at least an isothermal rectrotransciptase is comprised in a range between 2.0 and 4.5 units.

According to a preferred embodiment of the invention, the at least one cofactor is selected from the group consisting of: MgSO₄, 2-(trimethylazaniumyl)acetate, or mixtures thereof.

In one embodiment, the at least one cofactor is comprised at a concentration between 0.3 mM and 10 mM.

Suitable amplification solutions according to the invention are those capable of amplifying a single copy or a few copies of a piece of DNA across several orders of magnitude. In this case cDNA, because it was synthetized from an RNA template.

In an embodiment, the amplification solution comprises an amplification mixture comprising at least an isothermal polymerase, a deoxyribonucleotide triphosphate (dNTP) mixture and set of LAMP primers to amplify specific regions of *Legionella's* spp. 16S rRNA gene.

In one embodiment, the isothermal polymerase is comprised in a range of 10 to 14 units, the dNTP mixture at a concentration between 1.0 to 2.3 mM, and the set of LAMP primers to amplify specific regions of *Legionella's* spp. 16S rRNA gene in an average between 4,500 nM and 6,500 nM.

### Set of LAMP primers to amplify specific regions of Legionella's spp. 16S rRNA gene

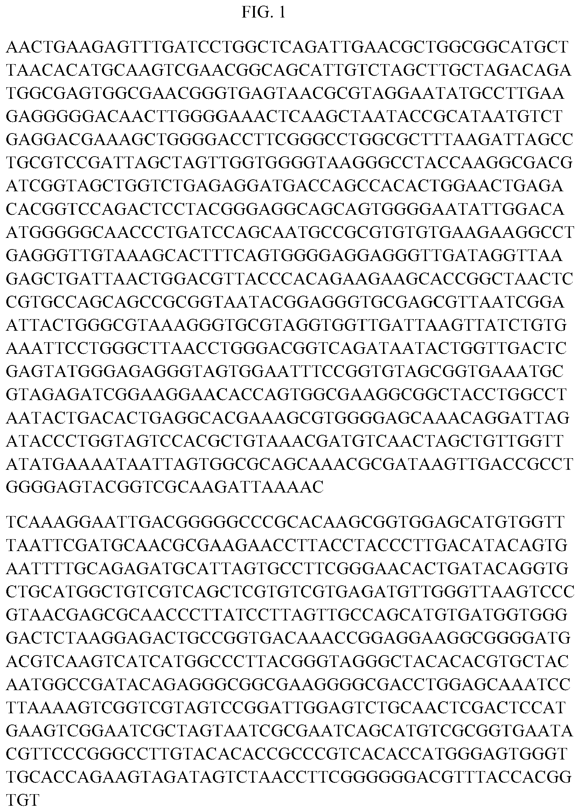

The specific LAMP primers have been designed on the basis of *Legionella pneumophila* subsp. *pneumophila* strain Philadelphia 1 16S ribosomal RNA gene, NCBI Reference Sequence: NR_074231.1>gi|444303809|ref|NR_074231.1| (FIG. 1). The primers architecture, was designed, taking into account in the sequence construction, the ones which present the greater polymorphism for the exclusive species; and at the same time, the greater homology, for the inclusive strains. Therefore, recognizing both, *Legionella pneumophila* and *Legionella* non pneumophila strains,

Any nucleic acid sequences listed herein or in the accompanying sequence listing are shown using standard letter abbreviations for nucleotide bases.

A minimum of eight primers were designed on the basis of a range between 180 and 400 base pairs:
Forward inner primer F3 comprising a nucleotide sequence as set forth in SEQ ID No. 1;
Forward inner primer F2 comprising a nucleotide sequence as set forth in SEQ ID No. 2;
Forward inner primer F1c comprising a nucleotide sequence as set forth in SEQ ID No. 3.
Backward inner primer B3 comprising a nucleotide sequence as set forth in SEQ ID No. 4;
Backward inner primer B2 comprising a nucleotide sequence as set forth in SEQ ID No. 5;
Backward inner primer B1c comprising a nucleotide sequence as set forth in SEQ ID No. 6;
Forward inner primer FIP comprising a nucleotide sequence as set forth in SEQ ID No. 7;
Backward inner primer BIP comprising a nucleotide sequence as set forth in SEQ ID No. 8.

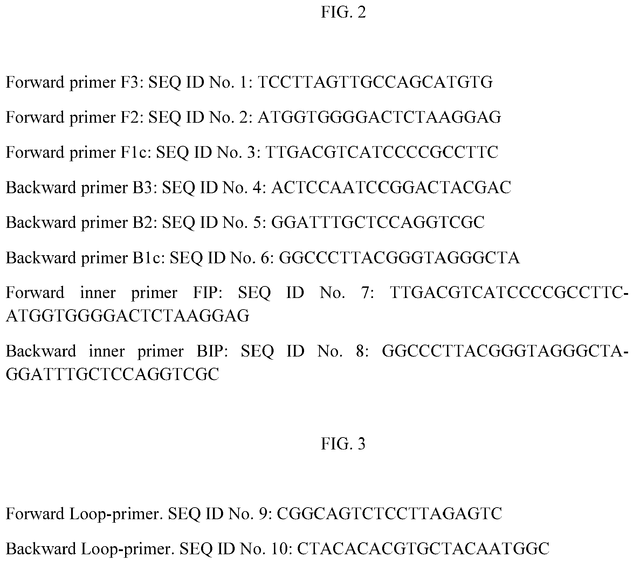

FIG. 2 shows the nucleotide sequences of the LAMP-primers according to the invention.

Thus, in one embodiment of the detection method according to the invention, the set of LAMP primers to amplify specific regions of *Legionella's* 16S rRNA gene comprises at least: a forward primer F3 comprising a nucleotide sequence as set forth in SEQ ID No. 1; a forward primer F2 comprising a nucleotide sequence as set forth in SEQ ID No. 2; a forward primer F1c comprising a nucleotide sequence as set forth in SEQ ID No. 3; a backward primer B3 comprising a nucleotide sequence as set forth in SEQ ID No. 4; a backward primer B2 comprising a nucleotide sequence as set forth in SEQ ID No. 5; a backward primer B1c comprising a nucleotide sequence as set forth in SEQ ID No. 6; a forward inner primer FIP comprising a nucleotide sequence as set forth in SEQ ID No. 7; and a backward inner primer BIP comprising a nucleotide sequence as set forth in SEQ ID No. 8.

In order to accelerate the molecular reactions another pair of "loop-primers" can be added.

Accordingly, in another embodiment that the set of LAMP primers to amplify specific regions of *Legionella's* 16S rRNA gene further comprises a forward loop-primer comprising a nucleotide sequence as set forth in SEQ ID No. 9 and a backward loop-primer comprising a nucleotide sequence as set forth in SEQ ID No 10 (See FIG. 3).

Along the isothermal amplification of the nucleic acids, the reaction yields high quantities of pirophosphate as by-product of the enzymatic reaction. This pyrophosphate reacts with the magnesium ions present in the reaction mixture. These complexes can be dyed with specific fluorophores, in the Frequency Amplitude Modulated (FAM) spectrum, which can develop a colour change in the reaction mixture, that can be detected in the visible spectrum at the same time that the reaction takes place. A direct reading of the reaction with naked eye is thus possible.

Accordingly, in a preferred embodiment of the invention, the reading solution comprises at least one reading reagent comprising a Frequency Amplitude Modulated (FAM) spectra fluorophore.

Suitable FAM spectra fluorophores according to the invention comprise, but are not limited to: calceine, hydroxyl naphthol blue (HNB), 6-carboxyfluorescein, Alexa fluor 488, Fluorescein FITC.

In one embodiment, the at least one reading reagent is comprised in a concentration between 25 and 75% reaction volume.

In yet a more preferred embodiment of the invention, the detection reagent comprising elution, rectrotranscriptase, amplification and reading buffers and/or reagents, and cofactors in step (c) comprises: 2.0 to 4.5 units of isothermal retrotranscriptase, 6.3 to 10 mM of MgSO₄ and 0.3 to 2.3 M of 2-(trimethylazaniumyl)acetate, 1.0 to 2.3 mM of dNTP mixture and in average between 4500 nM and 6500 nM of at least eight primers to amplify specific regions of *Legionella's* 16S rRNA gene; 10 to 14 units of isothermal Polymerase, a FAM-spectra fluophore in a concentration between 25 and 75% of the reaction volume, and 25 to 65%, in respect of the total volume of the solution: Elution, rectro-transcriptase, amplification and reading buffer, of Apyrogenic water.

### (d) Result reading

As already mentioned above, the use of a reading solution, in particular of specific fluorophores allows the direct reading of the reaction with naked eye.

### EXAMPLES

### Design and verification of the method for detection of Legionella spp. according to the invention

A novel design was done, and it included an in silico (BioEdit v5.0.6) study of the strains homology; it covered the isothermal amplification of 68 bacterial strains. 38 out of the 68, are considered by the invention as target inclusive strains (Table 1). These strains are reported by the World Health Organization (WHO), as responsible agents of Legionnaire's disease outbreaks (*Legionella* and the prevention of legionellosis". World Health Organization 2007). These strains, corresponding to different species within Legionella genus, are recognized by the invention as positive, 30 out of the 68, ought to be recognized as negative by the invention, in spite of their closeness in similar environments.

**Table 1: Inclusive species selected for the design of isothermal amplification in silicium**

| | Inclusive species | Strain | Serogroup |
|---|---|---|---|
| 1 | L. pneumophila | Philadelphia 1 | 1 |
| 2 | L. pneumophila | Knoxville-1 | 1 |
| 3 | L. pneumophila | CA1 | 1 |
| 4 | L. pneumophila | Alcoy | 1 |
| 5 | L. pneumophila | strain MDC1252 | 2 |
| 6 | L. pneumophila | strain E5C1 | 3 |
| 7 | L. pneumophila | Los Angelos-1 | 4 |
| 8 | L. pneumophila | Los Angelos-1 | 4 |
| 9 | L. pneumophila | strain U8W | 5 |
| 10 | L. pneumophila | Dallas - IE | 5 |
| 11 | L. pneumophila | JCM 7571 | 5 |
| 12 | L. pneumophila | ATCC 33215 | 6 |
| 13 | L. pneumophila | strain 4424 | 6 |
| 14 | L. pneumophila | strain MDC1257 | 7 |
| 15 | L. pneumophila | | 8 |
| 16 | L. pneumophila | strain GgN7 | 8 |
| 17 | L micdadei | TATLOCK | |
| 18 | L. dumoffii | NY 23 | |
| 19 | L. dumofii | A1.4F | |
| 20 | Legionella maceachernii | ATCC 35300 | |
| 21 | Legionella maceachernii | strain 4475 | |
| 22 | L. jordanis | BL-540 | |
| 23 | L. erythra | SE-32A-C8 | |
| 24 | L. tucsonensis | 1087-AZ-H | |
| 25 | Legionella bozemanii | WIGA | |
| 26 | L. anisa | 30_T1_200 7 | |
| 27 | L. anisa | ucsc16 | |
| 28 | L. longbeachae | Long-Beach-4 | 1 |
| 29 | L. longbeachae | ATCC 33484 | 2 |
| 30 | L. wadsworthii | NCTC 11532 | |
| 31 | L. hackeliae | Lansing-2 | |
| 32 | L. sainthelensi | NCTC 11988 | |
| 33 | L. feeleii | WO-44C | |
| 34 | L. birminghamensis | NCTC 12437 | |
| 35 | L. cincinnatiensis | NCTC 12438 | |
| 36 | L. lansingensis | NCTC 12830 | |
| 37 | L oakridgensis | KFYY-2 | |
| 38 | L. parisiensis | ATCC:700174 | |

**Table 2: Exclusive species selected for the design of isothermal amplification in silicium**

| | Exclusive species | Strain |
|---|---|---|
| 1 | Escherichia coli | |
| 2 | Enterococcus faecium | strain Aus0004 |
| 3 | Clostridium perfringens | strain 13 |
| 4 | Salmonella typhi | |
| 5 | Candida albicans | |
| 6 | Pseudomonas aeruginosa | Strain DSM 50071 |
| 7 | Staphylococcus aureus | N315 strain N315 |
| 8 | Proteus vulgaris | KCCM:1153 9 |
| 9 | Listeria monocytogenes | strain 07PF0776 |
| 10 | Enterobacter cloacae | ATCC 13047 |
| 11 | Serratia marcescens | strain WW4 |
| 12 | Caulobacter sp | strain K31 |
| 13 | Acinetobacter calcoaceticus | |
| 14 | Aeromonas hydrophila | strain CCM 7232 |
| 15 | Bacillus subtilis | BAB-1815 |
| 16 | Burkholderia cepacia | GG4 |
| 17 | Citrobacter freundii | DSM 30039 |
| 18 | Citrobacter koseri | ATCC BAA-895 |
| 19 | Klebsiella oxytoca | KCTC 1686 |
| 20 | Pseudomonas fluorescens | Pf0-1 |
| 21 | Pseudomonas putida | F1 |
| 22 | Pseudomonas stutzeri | phen8 |
| 23 | Ralstonia Pickettii | 12J |
| 24 | Raoultella terrigena | NBRC 14941 |
| 25 | Streptococcus pyrogenes | M1 GAS |
| 26 | Yersinia ruckeri | |
| 27 | Alcaligenes faecalis | |
| 28 | Enterobacter aerogenes | KCTC 2190 |
| 29 | Flavobacterium sp. | W1.09-205 |
| 30 | Stenotrophomonas maltophila | R551-3 |

Regarding the specific primers architecture, 18 different designs were done, taking into account in the sequence construction, the ones which present the greater polymorphism for the exclusive species; and at the same time, the greater homology, for the inclusive strains, which were identified as target among the Legionella's genus strains. Data shown in Figure 11, the loci for the specificity primers are remarked.

The inclusivity and exclusivity tests of the different primers designs were performed following the AFNOR standard: "Validation protocol for commercial methods of detection and quantification of Legionella and Legionella pneumophila by concentration and gene amplification by polymerase chain reaction (PCR) "; No.NF148 ; 30 October 2013). This standard is focused on the validation of PCR's commecial procedures, for the *Legionella* spp. and *Legionella pneumophila* detection.

The isothermal amplification conditions were optimised, taking into account the following parameters: reaction temperature, the cofactors concentration, the primers concentration,the enzymatic blends, and the wave length of the fluorescence emision. The assay control was performed by rt-PCR, and by the analysis of the amplification products by gel electrophoresis.

Table 3 shows the tested strains, for the specificity assays, which include: different serogroups belonging to *Legionella pneumophila;* as well as, the strains belonging to *Legionella* genus, but which are not pneumophila specie. And at the end of the Table, the strains defined for the exclusivity test, which are naturally occurring in water but which do not belong to *Legionella* spp.

**Table 3. Strains selected for the specificity test, belonging to Legionella spp; and non Legionella spp, which are closely related strains, in the water environmental niches.**

| Species | Serogroup | Strain | Expected Result |
|---|---|---|---|
| *L.pneumophila* | 1 | DMS 7513 *Philadelphia-1* | Positiv |
| *L.pneumophila* | 1 | DMS 25199 Benidorm | Positiv |
| *L.pneumophila* | 3 | DMS 2540 | Positiv |
| *L.pneumophila* | 6 | DMS25182 | Positiv |
| *L.pneumophila* | 2 | DMS25038 | Positiv |
| *L.pneumophila* | 4 | DMS7514 | Positiv |
| *L.pneumophila* | 5 | DMS24991 | Positiv |
| *L. micdadei* | | CCUG31229AT | Positiv |
| *L.bozemanii (Fluoribacter bozemanae)* | | DMS 16523 | Positiv |
| *L. dumofii (Fluoribacter dumofii)* | | DMS 17625 | Positiv |
| *L. longbeachae* | | DMS 10572 | Positiv |
| *Escherichia coli* | | CECT 4056 | Negativ |
| *Clastridium perfingenes* | | CECT 376 | Negativ |
| *P. aeruginosa* | | CECT 116 | Negativ |
| *Enterobacter aerogenes* | | CECT 684 | Negativ |

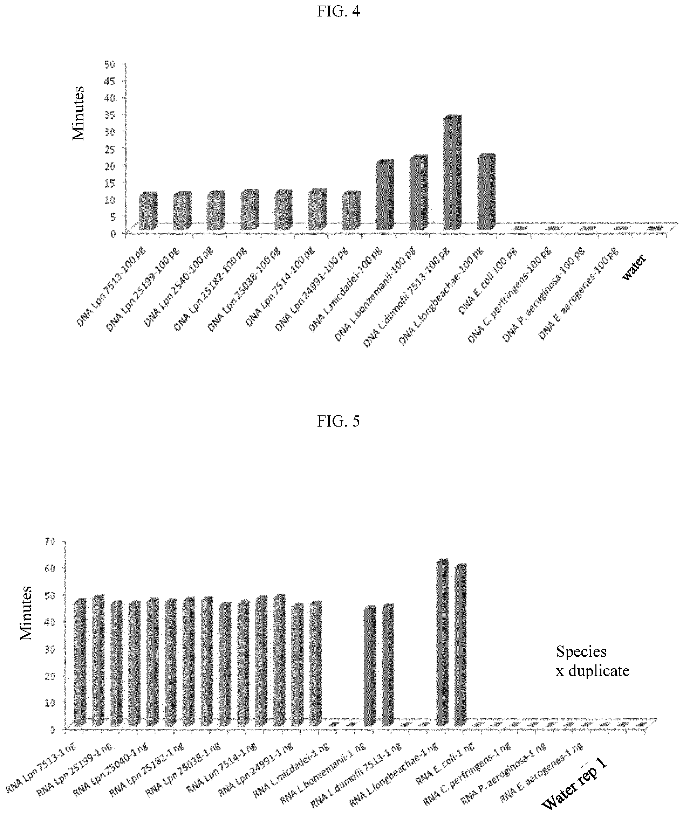

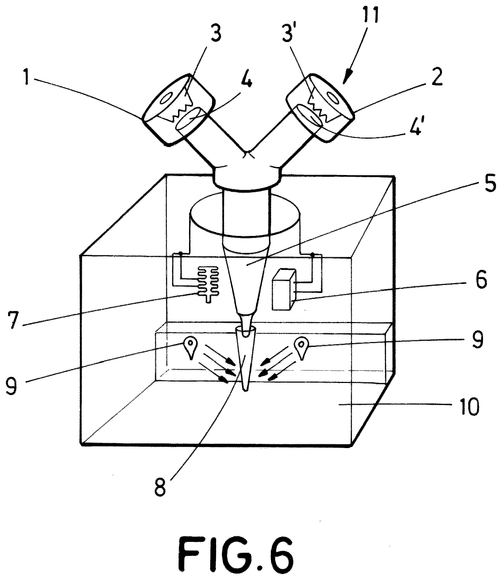

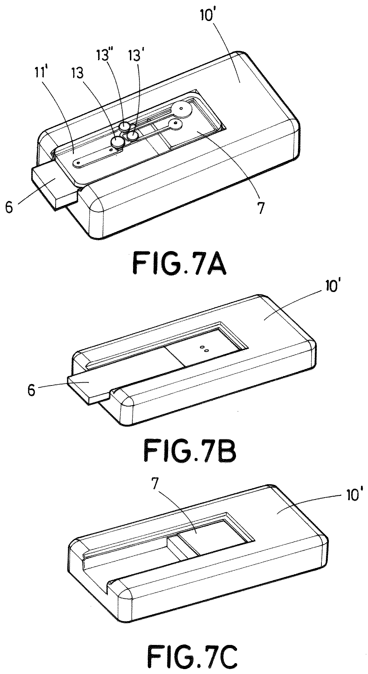

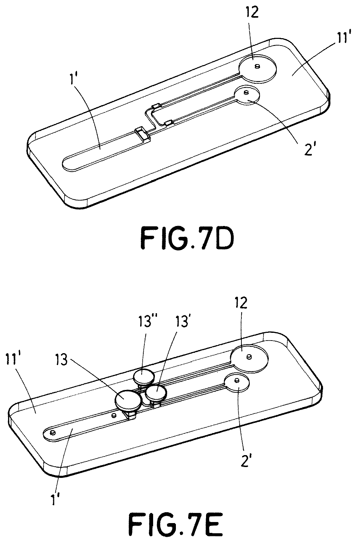

Figure 4 shows the specificity results of the described LAMP method. Assay performed with genome samples, calibrated with the set of primers specified by the present invention; results shown, the obtained data from quantification cycle (Cq), or time in minutes in which the fluorescence emission, becomes exponential.

Figure 5 shows specificity results of the described LAMP method. Assay performed with genome samples, calibrated with a comparative set of primers specified by the present invention; results shown, the obtained data from quantification cycle (Cq), or time in minutes in which the fluorescence emission, becomes exponential. Below, the fluorescence emission of each reaction tubes, at the assay final time.

As it can be shown in the above- mentioned figures (4 and 5), the set of primers according with the invention have a better performance than the other ones.

### Study of the sensibility threshold of the amplification test according to the invention

The analytical detection limit of the detection method according to the invention was determined based on calibrated RNA from the reference strain of *Legionella pneumophila* DSM 7513. As shown in table 4, the detection method according to the invention is capable of detection the equivalent to 1 pg of *Legionella's* RNA, which corresponds to a Genomic Equivalent (GE) of 10 CFU. The coefficient of correlation between the beginning of the fluorescence emission and the initial amount of genetic material was greater than 0.99 for four logarithmic orders.

**Table 4: Results obtained in the study of the threshold limit of the amplification test, for Legionella detection. Calibrated samples of RNA obtained from the reference strain was used**

| *DNA Legionella pneumophila* | Cq 1 | Cq 2 | Deviation |
|---|---|---|---|
| 1 ng (10000 GE) | 14.9 | 15.15 | 0.177 |
| 100 pg (1000 GE) | 18.32 | 18.07 | 0.177 |
| 10 pg (100 GE) | 22.12 | 21.57 | 0.389 |
| 1 pg (10 GE) | 25.4 | 25.31 | 0.064 |

Table 5 shows the results for the three concentrations established according to legislation: 1 ng (GE to 10,000 CFU), 100 pg (GE to 1,000 CFU) and 10 pg (GE to 100 CFU) per five samples to control the precision of the detection method according to the invention. As it can be seen the coefficient of variation is less than 1.2% for the three levels of *Legionella* (low, high and very high).

**Table 5: Study of the precision of the amplification test for Legionella detection. Calibrated samples of RNA obtained from the reference strain was used**

| *DNA Legionella pneumophila* | Cq 1 | Cq 2 | Cq3 | Cq4 | Cq5 | Deviation |
|---|---|---|---|---|---|---|
| 1 ng (10000 GE) | 14.78 | 14.55 | 14.53 | 14.43 | 14.26 | 0.163 |
| 100 pg (1000 GE) | 17.94 | 18.00 | 18.04 | 17.89 | 17.77 | 0.105 |
| 10 pg (100 GE) | 20.59 | 20.56 | 20.63 | 20.48 | 20.27 | 0.143 |

The sensitivity assays for the detection method according to the invention were performed for pure cultures of the reference strain of *Legionella pneumophila* DSM 7513. For these assays, the cultures were prepared in BCYE plaques (culture medium for *in vitro* growth of *Legionella* spp.), allowed to grow for 3 days at 37°C. Subsequently, the pure cultures were collected in RINGER medium for dilution to working concentrations corresponding to those stipulated by the legislation (10⁴, 10³ and 10²), and required by the market. The exact amount of *Legionella* analysed by the genetic test is also confirmed by the culture in plaques. Thus, the genomic extraction was optimized in combination with the isothermal amplification for obtaining maximum reproducibility, especially at lower bacterial concentration.

Several environmental samples from different facilities were tested with the detection method according to the invention and compared with the Gold standard ISO 11731 (see table 6).

**Table 6: Comparative results of detection method according to the invention and Gold Standard ISO 11731**

| Environmental Sample | Method of detection according to the invention | Gold Standard ISO 11731 test |
|---|---|---|
| M1 | Negative/litre | Negative/litre |
| M2 | Negative/litre | Negative/litre |
| M3 | Positive/650 ml | Negative (7.500 CFU)/litre |
| M4 | Positive/350 ml | Negative/litre |
| M5 | Negative/200 ml | Negative/litre |
| M6 | Negative/350 ml | Negative/litre |

Sample M4 had a high content of non-soluble iron. This did, however, not affect the detection method according to the invention, as shown in table 1, since *Legionella spp.* was unequivocally detected. The detection of *Legionella* spp. in the same sample by the Gold standard ISO11731 test was not possible, since the bacteria cannot be cultivated, probably because the iron acts as an *in vitro* growth inhibitor.

| *DNA Legionella pneumophila* | Cq 1 | Cq 2 | Deviation |
|---|---|---|---|
| 1 ng (10000 GE) | 14.9 | 15.15 | 0.177 |
| 100 pg (1000 GE) | 18.32 | 18.07 | 0.177 |
| 10 pg (100 GE) | 22.12 | 21.57 | 0.389 |
| 1 pg (10 GE) | 25.4 | 25.31 | 0.064 |

Table 5 shows the results for the three concentration established according to legislation: 1 ng (GE to 10,000 CFU), 100 pg (GE to 1,000 CFU) and 10 pg (GE to 100 CFU) per five samples to control the precision of the detection method according to the invention. As it can be seen the coefficient of variation is less than 1.2% for the three levels of *Legionella* (low, high and very high).

**Table 5: Study of the precision of the amplification test for Legionella detection. Calibrated samples of RNA obtained from the reference strain was used**

| *DNA Legionella pneumophila* | Cq 1 | Cq 2 | Cq3 | Cq4 | Cq5 | Deviation |
|---|---|---|---|---|---|---|
| 1 ng (10000 GE) | 14.78 | 14.55 | 14.53 | 14.43 | 14.26 | 0.163 |
| 100 pg (1000 GE) | 17.94 | 18.00 | 18.04 | 17.89 | 17.77 | 0.105 |
| 10 pg (100 GE) | 20.59 | 20.56 | 20.63 | 20.48 | 20.27 | 0.143 |

The sensitivity assays for the detection method according to the invention were performed for pure cultures of the reference strain of *Legionella pneumophila* DSM 7513. For these assays, the cultures were prepared in BCYE plaques (culture medium for *in vitro* growth of *Legionella* spp.), allowed to grow for 3 days at 37°C. Subsequnetly, the pure cultures were collected in RINGER medium for dilution to working concentrations corresponding to those estipulated by the legislation (10⁴, 10³ and 10²), and required by the market. The exact amount of *Legionella* analysed by the genetic test is also confirmed by the culture in plaques. Thus, the genomic extraction was optimized in combination with the isothermal amplification for obtaining maximum reproducibility, especially at lower bacterial concentration.

Several environmental samples from different facilities were tested with the detection method according to the invention and compared with the Gold standard ISO 11731 (see table 6).

**Table 6: Comparative results of detection method according to the invention and Gold Standard ISO 11731**

| Environmental Sample | Method of detection I according to the invention | Gold Standard ISO 11731 test |
|---|---|---|
| M1 | Negative/litre | Negative/litre |
| M2 | Negative/litre | Negative/litre |
| M3 | Positive/650 ml | Negative (7.500 CFU)/litre |
| M4 | Positive/350 ml | Negative/litre |
| M5 | Negative/200 ml | Negative/litre |
| M6 | Negative/350 ml | Negative/litre |

Sample M4 had a high content of non-soluble iron. This did, however, not affect the detection method according to the invention, as shown in table 1, since *Legionella spp.* was unequivocally detected. The detection of *Legionella* spp. in the same sample by the Gold standard ISO11731 test was not possible, since the bacteria cannot be cultivated, probably because the iron acts as an *in vitro* growth inhibitor.

## Claims

1. A method for the detection of *Legionella spp.* strains in an environmental sample based on loop-mediated isothermal amplification **characterized in that** it comprises the steps of:
(a) sample collection;
(b) genetic material extraction of the sample with an extraction reagent comprising at least cellular lysis reagents and a magnetizable support solution;
(c) elution of the genetic material from the magnetizable support, and isothermal RNA retrotranscription and cDNA amplification with a detection reagent comprising at least elution, rectrotranscriptase, amplification and reading solutions, wherein the amplification solution comprises a set of LAMP primers to amplify specific regions of *Legionella's* 16S rRNA gene; and
(d) result reading,
wherein the set of LAMP primers to amplify specific regions of *Legionella's* 16S rRNA gene in step (c) comprises at least:
a forward primer F3 comprising a nucleotide sequence as set forth in SEQ ID No. 1; a
forward primer F2 comprising a nucleotide sequence as set forth in SEQ ID No. 2; a
forward primer F1c comprising a nucleotide sequence as set forth in SEQ ID No. 3; a
backward primer B3 comprising a nucleotide sequence as set forth in SEQ ID No. 4; a
backward primer B2 comprising a nucleotide sequence as set forth in SEQ ID No. 5; a
backward primer B1c comprising a nucleotide sequence as set forth in SEQ ID No. 6; a
forward inner primer FIP comprising a nucleotide sequence as set forth in SEQ ID No. 7;
and a backward inner primer BIP comprising a nucleotide sequence as set forth in SEQ ID No. 8.

2. The method according to claim 1, **characterized in that** the isothermal RNA retrotranscription and cDNA amplification in step (c) take place simultaneously at a temperature between 58°C and 74°C.

3. The method according to any of claims 1 to 2, **characterized in that** the set of LAMP primers to amplify specific regions of *Legionella's* 16S rRNA gene in step (c) further comprises a forward loop-primer comprising a nucleotide sequence as set forth in SEQ ID No. 9 and a backward loop-primer comprising a nucleotide sequence as set forth in SEQ ID No 10.

4. The method according to any of claims 1 to 3, **characterized in that** the reading solution in step (c) comprises at least one reading reagent comprising a Frequency Amplitude Modulated (FAM) spectra fluorophore.

5. A detection reagent for specifically detecting sequences of *Legionella's* 16S rRNA gene, **characterized in that** it comprises at least elution, rectrotranscriptase, amplification and reading solutions, wherein the amplification solution comprises a set of LAMP primers to amplify specific regions of *Legionella's* 16S rRNA gene, and
wherein the set of LAMP primers to amplify specific regions of *Legionella's* 16S rRNA gene comprises at least: a forward primer F3 comprising a nucleotide sequence as set forth in SEQ ID No. 1; a forward primer F2 comprising a nucleotide sequence as set forth in SEQ ID No. 2; a forward primer F1c comprising a nucleotide sequence as set forth in SEQ ID No. 3; a backward primer B3 comprising a nucleotide sequence as set forth in SEQ ID No. 4; a backward primer B2 comprising a nucleotide sequence as set forth in SEQ ID No. 5; a backward primer B1c comprising a nucleotide sequence as set forth in SEQ ID No. 6; a forward inner primer FIP comprising a nucleotide sequence as set forth in SEQ ID No. 7; and a backward inner primer BIP comprising a nucleotide sequence as set forth in SEQ ID No. 8.

6. The detection reagent for specifically detecting sequences of *Legionella's* 16S rRNA gene according claim 5, **characterized in that** the set of LAMP primers to amplify specific regions of *Legionella's* 16S rRNA gene further comprises a forward loop-primer comprising a nucleotide sequence as set forth in SEQ ID No. 9 and a backward loop-primer comprising a nucleotide sequence as set forth in SEQ ID No 10.

7. A set of LAMP primers to amplify specific regions of *Legionella's* 16S rRNA gene, **characterized in that** it comprises at least comprises at least: a forward primer F3 comprising a nucleotide sequence as set forth in SEQ ID No. 1; a forward primer F2 comprising a nucleotide sequence as set forth in SEQ ID No. 2; a forward primer F1c comprising a nucleotide sequence as set forth in SEQ ID No. 3; a backward primer B3 comprising a nucleotide sequence as set forth in SEQ ID No. 4; a backward primer B2 comprising a nucleotide sequence as set forth in SEQ ID No. 5; a backward primer B1c comprising a nucleotide sequence as set forth in SEQ ID No. 6; a forward inner primer FIP comprising a nucleotide sequence as set forth in SEQ ID No. 7; and a backward inner primer BIP comprising a nucleotide sequence as set forth in SEQ ID No. 8.

8. The set of LAMP primers to amplify specific regions of *Legionella's* 16S rRNA gene according to claim 7, **characterized in that** it further comprises a forward loop-primer comprising a nucleotide sequence as set forth in SEQ ID No. 9 and a backward loop-primer comprising a nucleotide sequence as set forth in SEQ ID No 10.

## Patentansprüche

1. Verfahren zum Nachweisen von Stämmen von *Legionella spp.* in einer Umweltprobe auf der Grundlage von schleifenvermittelter isothermer Amplifikation, **dadurch gekennzeichnet, dass** es folgende Schritte umfasst:
(a) Probenahme;
(b) Extraktion von genetischem Material der Probe mit einem Extraktionsreagens, das mindestens zelluläre Lysereagenzien und eine magnetisierbare Trägerlösung umfasst;
(c) Elution des genetischen Materials vom magnetisierbaren Träger und isotherme RNA-Retrotranskription und cDNA-Amplifikation mit einem Nachweisreagens, das mindestens Elutions-, Rektrotranskriptase-, Amplifikations- und Leselösungen umfasst, wobei die Amplifikationslösung einen Satz von LAMP-Primern umfasst, um bestimmte Bereiche des 16S rRNA-Gens von *Legionella* zu amplifizieren; und
(d) Ergebnisablesung,
wobei der Satz von LAMP-Primern zum Amplifizieren bestimmter Bereiche des 16S rRNA-Gens von *Legionella* in Schritt (c) mindestens Folgendes umfasst:
einen Vorwärtsprimer F3, der eine Nukleotidsequenz gemäß SEQ ID Nr. 1 umfasst; einen Vorwärtsprimer F2, der eine Nukleotidsequenz gemäß SEQ ID Nr. 2 umfasst; einen Vorwärtsprimer F1c, der eine Nukleotidsequenz gemäß SEQ ID Nr. 3 umfasst; einen Rückwärtsprimer B3, der eine Nukleotidsequenz gemäß SEQ ID Nr. 4 umfasst; einen Rückwärtsprimer B2, der eine Nukleotidsequenz gemäß SEQ ID Nr. 5 umfasst; einen Rückwärtsprimer B1c, der eine Nukleotidsequenz gemäß SEQ ID Nr. 6 umfasst; einen inneren Vorwärtsprimer FIP, der eine Nukleotidsequenz gemäß SEQ ID Nr. 7 umfasst; und einen inneren Rückwärtsprimer BIP, der eine Nukleotidsequenz gemäß SEQ ID Nr. 8 umfasst.

2. Verfahren nach Anspruch 1, **dadurch gekennzeichnet, dass** die isotherme RNA-Retrotranskription und cDNA-Amplifikation in Schritt (c) gleichzeitig bei einer Temperatur zwischen 58 °C und 74 °C erfolgen.

3. Verfahren nach einem der Ansprüche 1 bis 2, **dadurch gekennzeichnet, dass** der Satz von LAMP-Primern zum Amplifizieren bestimmter Bereiche des 16S rRNA-Gens von *Legionella* in Schritt (c) ferner einen Vorwärtsschleifenprimer umfasst, der eine Nukleotidsequenz gemäß SEQ ID Nr. 9 umfasst, sowie einen Rückwärtsschleifenprimer, der eine Nukleotidsequenz gemäß SEQ ID Nr. 10 umfasst.

4. Verfahren nach einem der Ansprüche 1 bis 3, **dadurch gekennzeichnet, dass** die Leselösung in Schritt (c) mindestens ein Lesereagens umfasst, das ein Fluorophor mit frequenz-amplitudenmoduliertem (FAM) Spektrum umfasst

5. Nachweisreagens zum spezifischen Nachweisen von Sequenzen des 16S rRNA-Gens von *Legionella* , **dadurch gekennzeichnet, dass** es mindestens Elutions-, Rektrotranskriptase-, Amplifikations- und Leselösungen umfasst, wobei die Amplifikationslösung einen Satz von LAMP-Primern umfasst, um bestimmte Bereiche des 16S rRNA-Gens von *Legionella* zu amplifizieren, und
wobei der Satz von LAMP-Primern zum Amplifizieren bestimmter Bereiche des 16S rRNA-Gens von *Legionella* mindestens Folgendes umfasst: einen Vorwärtsprimer F3, der eine Nukleotidsequenz gemäß SEQ ID Nr. 1 umfasst: einen Vorwärtsprimer F2, der eine Nukleotidsequenz gemäß SEQ ID Nr. 2 umfasst; einen Vorwärtsprimer F1c, der eine Nukleotidsequenz gemäß SEQ ID Nr. 3 umfasst; einen Rückwärtsprimer B3, der eine Nukleotidsequenz gemäß SEQ ID Nr. 4 umfasst; einen Rückwärtsprimer B2, der eine Nukleotidsequenz gemäß SEQ ID Nr. 5 umfasst; einen Rückwärtsprimer B1c, der eine Nukleotidsequenz gemäß SEQ ID Nr. 6 umfasst; einen inneren Vorwärtsprimer FIP, der eine Nukleotidsequenz gemäß SEQ ID Nr. 7 umfasst; und einen inneren Rückwärtsprimer BIP, der eine Nukleotidsequenz gemäß SEQ ID Nr. 8 umfasst.

6. Nachweisreagenz zum spezifischen Nachweisen von Sequenzen des 16S rRNA-Gens von *Legionella* nach Anspruch 5, **dadurch gekennzeichnet, dass** der Satz von LAMP-Primern zum Amplifizieren bestimmter Bereiche des 16S rRNA-Gens von *Legionella* ferner einen Vorwärtsschleifenprimer umfasst, der eine Nukleotidsequenz gemäß SEQ ID Nr. 9 umfasst, sowie einen Rückwärtsschleifenprimer, der eine Nukleotidsequenz gemäß SEQ ID Nr. 10 umfasst.

7. Satz von LAMP-Primern zum Amplifizieren bestimmter Bereiche des 16S rRNA-Gens von *Legionella,* **dadurch gekennzeichnet, dass** er mindestens Folgendes umfasst: einen Vorwärtsprimer F3, der eine Nukleotidsequenz gemäß SEQ ID Nr. 1 umfasst; einen Vorwärtsprimer F2, der eine Nukleotidsequenz gemäß SEQ ID Nr. 2 umfasst; einen Vorwärtsprimer F1c, der eine Nukleotidsequenz gemäß SEQ ID Nr. 3 umfasst; einen Rückwärtsprimer B3, der eine Nukleotidsequenz gemäß SEQ ID Nr. 4 umfasst; einen Rückwärtsprimer B2, der eine Nukleotidsequenz gemäß SEQ ID Nr. 5 umfasst; einen Rückwärtsprimer B1c, der eine Nukleotidsequenz gemäß SEQ ID Nr. 6 umfasst; einen inneren Vorwärtsprimer FIP, der eine Nukleotidsequenz gemäß SEQ ID Nr. 7 umfasst; und einen inneren Rückwärtsprimer BIP, der eine Nukleotidsequenz gemäß SEQ ID Nr. 8 umfasst.

8. Satz von LAMP-Primern zum Amplifizieren bestimmter Bereiche des 16S rRNA-Gens von *Legionella* nach Anspruch 7, **dadurch gekennzeichnet, dass** er weiterhin einen Vorwärtsschleifenprimer umfasst, der eine Nukleotidsequenz gemäß SEQ ID Nr. 9 umfasst, sowie einen Rückwärtsschleifenprimer, der eine Nukleotidsequenz gemäß SEQ ID Nr. 10 umfasst.

## Revendications

1. Procédé de détection de souches de *Legionella spp.* dans un échantillon environnemental sur la base de l'amplification isotherme facilitée par boucle, **caractérisé en ce qu'**il comprend les étapes consistant à :
(a) la collecte d'échantillons ;
(b) l'extraction du matériel génétique de l'échantillon avec un réactif d'extraction comprenant au moins des réactifs de lyse cellulaire et une solution de support magnétisable ;
(c) l'élution du matériel génétique du support magnétisable, et rétrotranscription isotherme de l'ARN et amplification de l'ADNc avec un réactif de détection comprenant au moins des solutions d'élution, de rétrotranscriptase, d'amplification et de lecture, la solution d'amplification comprenant un jeu d'amorces LAMP pour amplifier des régions spécifiques du gène de l'ARNr 16S de la *Legionella* ; et
(d) la lecture des résultats,
dans lequel le jeu d'amorces LAMP pour amplifier des régions spécifiques du gène de l'ARNr 16S de la *Legionella* dans l'étape (c) comprend au moins :
une amorce directe F3 comprenant une séquence nucléotidique telle que présentée dans la SEQ ID N° 1 ; une amorce directe F2 comprenant une séquence nucléotidique telle que présentée dans la SEQ ID N° 2 ; une amorce directe F1c comprenant une séquence nucléotidique telle que présentée dans la SEQ ID N° 3 ; une amorce inverse B3 comprenant une séquence nucléotidique telle que présentée dans la SEQ ID N° 4 ; une amorce inverse B2 comprenant une séquence nucléotidique telle que présentée dans la SEQ ID N° 5 ; une amorce inverse B1c comprenant une séquence nucléotidique telle que présentée dans la SEQ ID N° 6 ; une amorce directe interne FIP comprenant une séquence nucléotidique telle que présentée dans la SEQ ID N° 7 ; et une amorce inverse interne BIP comprenant une séquence nucléotidique telle que présentée dans la SEQ ID N° 8.

2. Procédé selon la revendication 1, **caractérisé en ce que** la rétrotranscription isotherme de l'ARN et l'amplification de l'ADNc dans l'étape (c) ont lieu simultanément à une température comprise entre 58 °C et 74 °C.

3. Procédé selon l'une quelconque des revendications 1 à 2, **caractérisé en ce que** le jeu d'amorces LAMP pour amplifier des régions spécifiques du gène de l'ARNr 16S de la *Legionella* à l'étape (c) comprend en outre une amorce boucle directe comprenant une séquence nucléotidique telle que présentée dans la SEQ ID N° 9 et une amorce boucle inverse comprenant une séquence nucléotidique telle que présentée dans la SEQ ID N° 10.

4. Procédé selon l'une quelconque des revendications 1 à 3, **caractérisé en ce que** la solution de lecture de l'étape (c) comprend au moins un réactif de lecture comprenant un fluorophore à spectre modulé en amplitude et fréquence (FAM).

5. Réactif de détection pour détecter spécifiquement des séquences du gène de l'ARNr 16S de la *Legionella,* **caractérisé en ce qu'il** comprend au moins des solutions d'élution, de rétrotranscriptase, d'amplification et de lecture, la solution d'amplification comprenant un jeu d'amorces LAMP pour amplifier des régions spécifiques du gène de l'ARNr 16S de la *Legionella,* et le jeu d'amorces LAMP pour amplifier des régions spécifiques du gène de l'ARNr 16S de la *Legionella* comprenant au moins : une amorce directe F3 comprenant une séquence nucléotidique telle que présentée dans la SEQ ID N° 1 ; une amorce directe F2 comprenant une séquence nucléotidique telle que présentée dans la SEQ ID N° 2 ; une amorce directe F1c comprenant une séquence nucléotidique telle que présentée dans la SEQ ID N° 3 ; une amorce inverse B3 comprenant une séquence nucléotidique telle que présentée dans la SEQ ID N° 4 ; une amorce inverse B2 comprenant une séquence nucléotidique telle que présentée dans la SEQ ID N° 5 ; une amorce inverse B1c comprenant une séquence nucléotidique telle que présentée dans la SEQ ID N° 6 ; une amorce directe interne FIP comprenant une séquence nucléotidique telle que présentée dans la SEQ ID N° 7 ; et un amorce inverse interne BIP comprenant une séquence nucléotidique telle que présentée dans la SEQ ID N° 8.

6. Réactif de détection pour détecter spécifiquement des séquences du gène de l'ARNr 16S de la *Legionella* selon la revendication 5, **caractérisé en ce que** le jeu d'amorces LAMP pour amplifier des régions spécifiques du gène de l'ARNr 16S de la *Legionella* comprend en outre une amorce boucle directe comprenant une séquence nucléotidique telle que présentée dans la SEQ ID N° 9 et une amorce boucle inverse comprenant une séquence nucléotidique telle que présentée dans la SEQ ID N° 10.

7. Jeu d'amorces LAMP pour amplifier des régions spécifiques du gène de l'ARNr 16S de la *Legionella,* **caractérisé en ce qu'il** comprend au moins : une amorce directe F3 comprenant une séquence nucléotidique telle que présentée dans la SEQ ID N° 1 ; une amorce directe F2 comprenant une séquence nucléotidique telle que présentée dans la SEQ ID N° 2 ; une amorce directe F1c comprenant une séquence nucléotidique telle que présentée dans la SEQ ID N° 3 ; une amorce inverse B3 comprenant une séquence nucléotidique telle que présentée dans la SEQ ID N° 4 ; une amorce inverse B2 comprenant une séquence nucléotidique telle que présentée dans la SEQ ID N° 5 ; une amorce inverse B1c comprenant une séquence nucléotidique telle que présentée dans la SEQ ID N° 6 ; une amorce directe interne FIP comprenant une séquence nucléotidique telle que présentée dans la SEQ ID N° 7 ; et une amorce inverse interne BIP comprenant une séquence nucléotidique telle que présentée dans la SEQ ID N° 8.

8. Jeu d'amorces LAMP pour amplifier des régions spécifiques du gène de l'ARNr 16S de la *Legionella* selon la revendication 7, **caractérisé en ce qu'**il comprend en outre une amorce boucle directe comprenant une séquence nucléotidique telle que présentée dans la SEQ ID N° 9 et un amorce boucle inverse comprenant une séquence nucléotidique telle que présentée dans la SEQ ID N° 10.
